(19) **Europäisches Patentamt**
**European Patent Office**
**Office européen des brevets**

(11) **EP 4 666 873 A1**

(12) **EUROPEAN PATENT APPLICATION**
published in accordance with Art. 153(4) EPC

(43) Date of publication:
**24.12.2025 Bulletin 2025/52**

(21) Application number: **24756982.5**

(22) Date of filing: **15.02.2024**

(51) International Patent Classification (IPC):
**A23L 33/185** $^{(2016.01)}$ **C07K 1/12** $^{(2006.01)}$
**C12P 21/00** $^{(2006.01)}$

(52) Cooperative Patent Classification (CPC):
**A23L 33/185; C07K 1/12; C12P 21/00**

(86) International application number:
**PCT/JP2024/005385**

(87) International publication number:
**WO 2024/172142 (22.08.2024 Gazette 2024/34)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **15.02.2023 JP 2023021612**
**28.04.2023 JP 2023074259**

(71) Applicant: **Amano Enzyme Inc.**
**Nagoya-shi**
**Aichi 460-8630 (JP)**

(72) Inventor: **SAKAI, Kiyota**
**Kakamigahara-shi, Gifu 509-0109 (JP)**

(74) Representative: **Vossius & Partner**
**Patentanwälte Rechtsanwälte mbB**
**Siebertstraße 3**
**81675 München (DE)**

(54) **PRODUCTION METHOD FOR PROCESSED VEGETABLE PROTEIN-CONTAINING LIQUID COMPOSITION**

(57)     The purpose of the present invention is to provide a processing technology that improves the solubility and/or digestibility of a protein in a plant protein-containing liquid composition. The solubility, digestibility, and/or mineral solubility of a protein in a plant protein-containing liquid composition can be improved by treating the plant protein-containing liquid composition with a protein deamidase and a cellulase.

**EP 4 666 873 A1**

**Description**

Technical Field

The present invention relates to a method for producing a processed plant protein-containing liquid composition. More specifically, the present invention relates to a method for enhancing solubility, digestibility, and/or mineral solubility of a protein in a plant protein-containing liquid composition.

Background Art

Beverages rich in nutrients such as proteins have been widely accepted by people since the nutrients can be easily taken in. On the other hand, plant milk prepared from a plant material abundantly containing a plant protein has been widely used as an alternative to animal milk typified by milk on the basis of an increase in the number of vegetarians, allergy problems, religious reasons, and the like in recent years.

Generally, plant milk has a problem of a low protein content unlike animal milk. Therefore, processing that improves protein solubility of the plant protein-containing liquid composition of plant milk is desired. For example, PTL 1 discloses a protein-containing oat based production method of allowing amylase, β-amylase, and a protein deamidase to act on oats. PTL 2 discloses a method for producing an oat protein composition having a protein content of 50% or more by allowing a protein deamidase to act on oats. PTL 3 discloses a method for producing a processed plant protein-containing liquid composition having improved solubility by treating a plant protein-containing liquid composition with a protease and a protein deamidase.

It is also known that a plant protein is inferior in digestibility to an animal protein (NPL 1), and a gap between food intake amount and nutrient absorption amount is also a problem.

Citation List

Patent Literature

PTL 1: WO 2014/123466 A
PTL 2: WO 2022/144452 A
PTL 3: WO 2022/102723 A

Non Patent Literature

NPL 1: Journal of Japan Society of Nutrition and Food Science, Vol. 20, No. 4, p. 259-266

Summary of Invention

Technical Problem

The plant protein-containing liquid composition can be treated with a protein deamidase to improve protein solubility. On the other hand, in view of the recent rapid spread and expansion of plant protein food and drink products, a technology that further enhances protein solubility is desired. Although the protein solubility can be improved by treating the plant protein-containing liquid composition with a protein deamidase and a protease, it is considered that if the solubility can be improved without hydrolysis of the protein, the characteristics (for example, mouthfeel) provided by the protein itself are more easily enjoyed. Alternatively, it is desired that the food characteristics such as digestibility and/or mineral solubility can be improved such that proteins and/or minerals contained in the plant protein-containing liquid composition can be taken into the body more efficiently.

It is therefore an object of the present invention to provide a novel processing technology that improves solubility, digestibility, and/or mineral solubility of a protein in a plant protein-containing liquid composition.

As a result of intensive studies, the present inventors have found that the solubility, digestibility, and mineral solubility of a protein can be improved by treating a plant protein-containing liquid composition with a protein deamidase and cellulase, compared with the case where the plant protein-containing liquid composition is treated with the protein deamidase alone. The present invention has been completed by further conducting studies based on these findings.

That is, the present invention provides inventions of the following aspects.

Item 1. A method for producing a processed plant protein-containing liquid composition, the method including treating a plant protein-containing liquid composition with a protein deamidase and cellulase.

Item 2. The method for producing a processed plant protein-containing liquid composition according to item 1, in which the plant protein is a cereal protein.

Item 3. The method for producing a processed plant protein-containing liquid composition according to item 1 or 2, in which the plant protein is an oat protein.

Item 4. The method according to any one of items 1 to 3, in which the cellulase is a cellulase from the genus Aspergillus and/or cellulase from the genus Trichoderma.

Item 5. The method according to any one of items 1 to 4, in which in the cellulase, exoglucanase activity is higher than endoglucanase activity.

Item 6. The method for producing a processed plant protein-containing liquid composition according to any one of items 1 to 5, in which the cellulase is used in an amount of 2 to 5000 U per U of the protein deamidase.

Item 7. The method for producing a processed plant protein-containing liquid composition according to any one of items 1 to 6, in which the protein deamidase is used in an amount of 0.01 to 200 U per g of the plant protein.

Item 8. A method for improving solubility, digestibility, and/or mineral solubility of a protein in a plant protein-containing liquid composition, the method including treating the plant protein-containing liquid composition with a protein deamidase and cellulase.

Item 9. An improver for solubility, digestibility, and/or mineral solubility of a protein in a plant protein-containing liquid composition, the improver containing a protein deamidase and a cellulase.

Item 10. The improver for solubility, digestibility, and/or mineral solubility of a protein according to item 9, the improver containing 2 to 5000 U of the cellulase per U of the protein deamidase.

Item 11. An improver for solubility, digestibility, and/or mineral solubility of a protein in a plant protein-containing liquid composition treated with a protein deamidase, the improver containing a cellulase.

Item 12. A plant protein-containing food and drink product including a processed plant protein-containing liquid composition obtained by the method according to any one of items 1 to 7.

Advantageous Effects of Invention

According to the present invention, there is provided a novel processing technology that improves solubility, digestibility, and/or mineral solubility of a protein in a plant protein-containing liquid composition.

Description of Embodiments

1. Method for Producing Processed Plant Protein-containing Liquid Composition

The method for producing a processed plant protein-containing liquid composition according to the present invention includes treating a plant protein-containing liquid composition with a protein deamidase and cellulase (enzyme treatment step). Since the solubility, digestibility, and/or mineral (at least one of calcium, iron, zinc, and magnesium; the same applies hereinafter) solubility of the protein in the plant protein-containing liquid composition can be improved in the enzyme treatment step, a plant protein-containing liquid composition processed to improve the solubility, digestibility, and/or mineral solubility of the protein is obtained. Hereinafter, the method for producing a processed plant protein-containing liquid composition according to the present invention will be described in detail.

1-1. Enzyme Treatment Step

In the enzyme treatment step, the plant protein-containing liquid composition is treated with a protein deamidase and cellulase.

1-1-1. Plant Protein-containing Liquid Composition

The plant protein-containing liquid composition used in the present invention is not particularly limited as long as it is a liquid containing a plant protein and cellulose in water. Specific examples of the plant protein-containing liquid composition include (i) a liquid obtained by dispersing in water a dry powder of at least one of a plant organ of a plant protein-derived plant and a material in which at least a part of components other than the protein and/or cellulose from the plant organ is, for example, removed to increase a content of the protein and/or cellulose (hereinafter referred to as a "plant protein material"), and if necessary, removing impurities derived from the peel or the like of the plant protein material by any means such as centrifugation, filtration, bag filtration, and sieving; (ii) a liquid obtained by crushing and dispersing a plant protein material in water, and if necessary, removing impurities derived from the peel or the like of the plant protein material by any means such as centrifugation, filtration, bag filtration, and sieving; (iii) a liquid obtained by, for example, removing at least one of the components other than the plant protein and/or cellulose from the liquid of (i) or (ii) to increase the content of the protein and/or cellulose; and (iv) a liquid obtained by mixing a dry powder prepared from the liquid of any one of (i) to (iii) with water. Preferable examples of the plant protein-containing liquid composition include plant milk.

In the following description, the "content of the plant protein material" in the plant protein-containing liquid composition is a proportion of the dry weight of the components constituting the plant organ contained in the plant protein-containing liquid composition. For example, when the plant protein-containing liquid composition is a liquid composed only of components derived from plant organs and water as in the liquids (i) to (iv), the "content of the plant protein material" refers to the proportion of the dry weight of the liquid. For example, when the plant protein-containing liquid composition is a liquid containing the liquids (i) to (iv) and an additive, the "content of the plant protein material" refers to the proportion of the dry weight of the portion of the liquid excluding the additive.

Examples of the plant protein material include, but are not particularly limited to, beans such as soybean, pea, lentil, chickpea, black bean, broad bean, mung bean, lupin bean, and kidney bean; cereals such as wheat, barley, oats, sorghum, rice, rye, buckwheat, Japanese millet, foxtail millet, teff, corn, and potato; nuts such as almond, coconut, peanut, cashew nut, hazelnut, pecan nut, macadamia nut, pistachio, walnut, brazil nut, pili nut, chestnut, sesame, and pine nut; and seeds such as hemp seed (industrial hemp), chia seed, chia, amaranthus, canary seed, and linseed.

In the present invention, the plant protein material may be used singly or in combination with a plurality types thereof. Among them, the plant protein material preferably includes cereals, more preferably wheat, barley, oat, sorghum, rice, rye, buckwheat, Japanese millet, foxtail millet, teff, corn, and potato, and still more preferably oats.

Examples of the content of the plant protein in the plant protein-containing liquid composition include, but are not particularly limited to, 0.01 to 50 wt%, 0.05 to 40 wt%, and 0.1 to 20 wt%, preferably 0.25 to 10 wt% and 0.5 to 5.0 wt%, and more preferably 1.0 to 2.0 wt% and 1.2 to 1.8 wt%.

Examples of the content of the cellulose in the plant protein-containing liquid composition include, but are not particularly limited to, 0.005 to 5 wt%, 0.01 to 4 wt%, and 0.05 to 3 wt%, preferably 0.1 to 2.5 wt% and 0.3 to 2 wt%, more preferably 0.5 to 1.5 wt%, and still more preferably 0.9 to 1.3 wt% in terms of the content of dietary fiber.

Examples of the content of the plant protein material in the plant protein-containing liquid composition include, but are not particularly limited to, 0.05 to 50 wt%, 0.1 to 40 wt%, and 0.5 to 30 wt%, preferably 1 to 25 wt% and 3 to 20 wt%, and more preferably 5 to 15 wt% and 10 to 13 wt%.

When the plant protein material contained in the plant protein-containing liquid composition is a starchy material such as grain (preferably, oat), such a plant protein-containing liquid composition is preferably subjected to an amylase treatment described later in "1-2-2. Amylase Treatment Step". Examples of the starch content in the plant protein-containing liquid composition before the amylase treatment step include 0.035 to 35 wt%, 0.07 to 28 wt%, and 0.35 to 21 wt%, preferably 0.7 to 18 wt% and 2 to 14 wt%, and more preferably 4 to 10 wt%.

1-1-2. Protein Deamidase

The protein deamidase used in the present invention is an enzyme that exhibits an action of decomposing an amide group-containing side chain of a protein without cleavage of a peptide bond and without crosslinking of the protein, and the type, origin, and the like thereof are not particularly limited.

Examples of the protein deamidase include an enzyme that deamidates a glutamine residue in a protein and converts it into glutamic acid (for example, protein glutaminase) and an enzyme that deamidates an asparagine residue in a protein and converts it into aspartic acid (for example, protein asparaginase). These protein deamidases may be used alone or in combination with two or more thereof. Among these protein deamidases, protein glutaminase is preferred from the viewpoint of further enhancing the effect of improving solubility, digestibility, and/or mineral solubility of the protein.

More specific examples of the protein deamidase include protein deamidases from the genus Chryseobacterium, the genus Flavobacterium, the genus Empedobacter, the genus Sphingobacterium, the genus Aureobacterium, the genus Myroides, the genus Luteimicrobium, the genus Agromyces, the genus Microbacterium, and the genus Leifsonia. Such a protein deamidase is known, and for example, JP 2000-50887 A, JP 2001-218590 A, WO 2006/075772 A1, and WO 2015/133590 A can be referred to.

The protein deamidase may be a natural protein deamidase or a recombinant protein deamidase.

These protein deamidases may be used singly or in combination with a plurality types thereof. Among these protein deamidases, from the viewpoint of further enhancing the effect of improving solubility, digestibility, and/or mineral solubility of the protein, the natural protein deamidase is preferably a protein deamidases from the genus Chryseobacterium, more preferably a protein glutaminase from the genus Chryseobacterium, still more preferably a protein glutaminase from Chryseobacterium proteolyticum sp., and even more preferably a protein glutaminase from the strain Chryseobacterium proteolyticum 9670.

The protein deamidase can be prepared from a culture solution of a microorganism from which the protein deamidase is derived or a transformant into which a gene related to the recombinant protein deamidase is incorporated. Specific examples of the preparation method include a method of recovering a protein deamidase from a culture solution or a bacterial cell of the microorganism. For example, in the case of using a microorganism that secretes a protein deamidase, the enzyme can be separated and/or purified after recovering bacterial cells from the culture solution in advance by filtration, centrifugation or the like, if necessary. In addition, in the case of using a microorganism that does not secrete a protein deamidase, after bacterial cells are recovered from the culture solution in advance, if necessary, the bacterial cells are disrupted by pressurization treatment, ultrasonic treatment, or the like to expose the enzyme, and then the enzyme can be separated and/or purified. As an enzyme separation and/or purification method, a known protein separation and/or purification method can be used without particular limitation, and examples thereof include a centrifugal separation method, a UF concentration method, a salting-out method, and various chromatography methods using an ion exchange resin or the like. The separated and/or purified enzyme can be pulverized by a drying method such as freeze-drying or reduced-pressure drying and can also be pulverized using an appropriate excipient and/or drying aid in the drying method. The separated and/or purified enzyme can also be liquefied by adding an appropriate additive and subjecting the mixture to filtration sterilization.

The amount of protein deamidase used is not particularly limited, but the amount used per g of the plant protein is, for example, 0.01 U or more. From the viewpoint of further enhancing the effect of improving solubility, digestibility, and/or mineral solubility of a protein, the amount of the protein deamidase used per g of the plant protein is preferably 0.1 U or more or 0.2 U or more, more preferably 0.5 U or more, still more preferably 1 U or more or 3 U or more, even more preferably 6 U or more, and still even more preferably 8 U or more.

The upper limit of the range of the amount of the protein deamidase used per g of the plant protein is not particularly limited, and examples thereof include 200 U or less, 100 U or less, 50 U or less, 30 U or less, 20 U or less, 15 U or less, and 12 U or less.

The amount of protein deamidase used per g of the plant protein material (in terms of dry weight) is, for example, 0.0013 U or more. From the viewpoint of further enhancing the effect of improving solubility, digestibility and/or mineral solubility of a protein, the amount of protein deamidase used per g of the plant protein material (in terms of dry weight) is preferably 0.013 U or more or 0.026 U or more, more preferably 0.065 U or more, still more preferably 0.13 U or more or 0.4 U or more, even more preferably 0.8 U or more, and still even more preferably 1 U or more.

The upper limit of the range of the amount of protein deamidase used per g of the plant protein material (in terms of dry weight) is not particularly limited, and examples thereof include 26 U or less, 13 U or less, 6.5 U or less, 4 U or less, 2.6 U or less, 2 U or less, and 1.5 U or less.

For the activity of the protein deamidase, the amount of enzyme that liberates 1 $\mu$mol of ammonia per minute using benzyloxycarbonyl-L-glutaminylglycine (Z-Gln-Gly) as a substrate is defined as 1 unit (1 U).

1-1-3. Cellulase

The cellulase used in the present invention is an enzyme that hydrolyzes a glucoside bond of $\beta$-1,4 glucan, and the type, origin, and the like thereof are not particularly limited.

Examples of the cellulase include an endoglucanase and an exoglucanase. These cellulases may be used singly or in combination with two or more thereof. Although all of these cellulases can improve protein solubility, from the viewpoint of further enhancing the effect of improving protein solubility, a cellulase containing at least an exoglucanase (that is, a cellulase consisting of exoglucanase or a cellulase containing an exoglucanase and an endoglucanase) is preferred, a cellulase mainly containing an exoglucanase (that is, a cellulase in which the exoglucanase activity is higher than the endoglucanase activity) is more preferred, and an exoglucanase is even more preferred. Although all of these cellulases can improve protein digestibility, from the viewpoint of further enhancing the effect of improving protein digestibility, a cellulase containing at least an exoglucanase (that is, a cellulase consisting of exoglucanase or a cellulase containing an exoglucanase and an endoglucanase) is preferred, a cellulase mainly containing an exoglucanase (that is, a cellulase in which the exoglucanase activity is higher than the endoglucanase activity) is more preferred, and an exoglucanase is even more preferred. Furthermore, although all of these cellulases can improve mineral solubility, from the viewpoint of further enhancing the solubility of zinc and magnesium, a cellulase containing at least an exoglucanase (that is, a cellulase consisting of exoglucanase or a cellulase containing an exoglucanase and an endoglucanase) is preferred, a cellulase mainly containing an exoglucanase (that is, a cellulase in which the exoglucanase activity is higher than the endoglucanase activity) is more preferred, and an exoglucanase is even more preferred.

Specific examples of the cellulase include cellulases derived from microorganisms, and more specific examples thereof include cellulases from the genus Aspergillus, the genus Trichoderma, and the genus Acremonium.

More specifically, examples of the cellulase from the genus Aspergillus include cellulases from Aspergillus niger, Aspergillus oryzae, Aspergillus sojae, Aspergillus saitoi, Aspergillus awamori, and Aspergillus flavus; examples of the cellulase from the genus Trichoderma include cellulases from Trichoderma reesei and Trichoderma viride; and examples of the cellulase from the genus Acremonium include cellulases from Acremonium cellulolyticus.

The cellulase may be a natural cellulase or a recombinant cellulase.

These cellulases may be used singly or in combination with a plurality types thereof. Among these cellulases, from the viewpoint of further enhancing the effect of improving the protein solubility, the natural cellulase preferably includes a cellulase from the genus Aspergillus and a cellulase derived from the genus Trichoderma, more preferably a cellulase from Aspergillus niger and a cellulase from Trichoderma viride. Among these cellulases, from the viewpoint of further enhancing the effect of improving the protein digestibility, the natural cellulase preferably includes a cellulase from the genus Aspergillus and a cellulase from the genus Trichoderma, more preferably a cellulase from the genus Trichoderma, and still more preferably a cellulase from Trichoderma viride. Furthermore, among these cellulases, the natural cellulase preferably includes a cellulases from the genus Aspergillus and a cellulase from the genus Trichoderma from the viewpoint of further enhancing the effect of improving the mineral solubility; from the viewpoint of further enhancing the solubility of zinc and magnesium, more preferably a cellulase from the genus Trichoderma, and still more preferably a cellulases from Trichoderma viride; and from the viewpoint of comprehensively enhancing the solubility of calcium, iron, zinc, and magnesium, more preferably a combination of a cellulase from the genus Aspergillus and a cellulase from the genus Trichoderma, and still more preferably a combination of a cellulase from Aspergillus niger and a cellulase from Trichoderma viride.

The cellulase can be prepared from a culture solution of a microorganism from which the cellulase is derived or a transformant into which a gene related to the recombinant cellulase is incorporated. Specific examples of the preparation method include a method of recovering a cellulase from a culture solution or a bacterial cell of the microorganism. For example, in the case of using a cellulase secreting type microorganism, the enzyme can be separated and/or purified after recovering bacterial cells from the culture solution in advance by filtration, centrifugation or the like, if necessary. In addition, in the case of using a non-cellulase secreting type microorganism, after bacterial cells are recovered from the culture solution in advance, if necessary, the bacterial cells are disrupted by pressurization treatment, ultrasonic treatment, or the like to expose the enzyme, and then the enzyme can be separated and/or purified. As an enzyme separation and/or purification method, a known protein separation and/or purification method can be used without particular limitation, and examples thereof include a centrifugal separation method, a UF concentration method, a salting-out method, and various chromatography methods using an ion exchange resin or the like. The separated and/or purified enzyme can be pulverized by a drying method such as freeze-drying or reduced-pressure drying and can also be pulverized using an appropriate excipient and/or drying aid in the drying method. The separated and/or purified enzyme can also be liquefied by adding an appropriate additive and subjecting the mixture to filtration sterilization.

The amount of cellulase used is not particularly limited, but the amount used per g of dietary fiber is, for example, 1 U or more. From the viewpoint of further enhancing the effect of improving solubility, digestibility, and/or mineral solubility of the protein, the amount used per g of dietary fiber is preferably 3 U or more, 6 U or more, 10 U or more, 13 U or more, 17 U or more, 20 U or more, 30 U or more, 40 U or more, 50 U or more, 70 U or more, 100 U or more, 300 U or more, 400 U or more, 500 U or more, 800 U or more, 1000 U or more, or 1300 U or more.

The upper limit of the range of the amount of the cellulase used per g of dietary fiber is not particularly limited, and examples thereof include 6000 U or less, 5000 U or less, 4000 U or less, 3000 U or less, 2000 U or less, 1700 U or less, 1500 U or less, 1200 U or less, 900 U or less, 600 U or less, 400 U or less, 200 U or less, 90 U or less, 80 U or less, 60 U or less, 50 U or less, 40 U or less, 30 U or less, 20 U or less, 15 U or less, 8 U or less, and 4 U or less.

The amount of cellulase used per g of the plant protein material (in terms of dry weight) is, for example, 0.1 U or more. From the viewpoint of further enhancing the effect of improving solubility, digestibility, and/or mineral solubility of the protein, the amount of cellulase used per g of the plant protein material (in terms of dry weight) is preferably 0.3 U or more, 0.6 U or more, 1 U or more, 1.3 U or more, 1.7 U or more, 2 U or more, 3 U or more, 4 U or more, 5 U or more, 7 U or more, 10 U or more, 30 U or more, 40 U or more, 70 U or more, 100 U or more, or 130 U or more. The upper limit of the range of the amount of the cellulase used per g of the plant protein material (in terms of dry weight) is not particularly limited, and examples thereof include 600 U or less, 500 U or less, 400 U or less, 300 U or less, 200 U or less, 170 U or less, 150 U or less, 120 U or less, 90 U or less, 60 U or less, 40 U or less, 20 U or less, 9 U or less, 8 U or less, 6 U or less, 5 U or less, 4 U or less, 3 U or less, 2 U or less, 1.5 U or less, 0.8 U or less, or 0.4 U or less.

The use ratio of the protein deamidase to the cellulase is determined based on the amount of each enzyme used, and examples of the amount of the cellulase used per U of the protein deamidase preferably include 0.1 U or more. From the viewpoint of further enhancing the effect of improving solubility, digestibility, and/or mineral solubility of the protein, the amount of the cellulase used per U of the protein deamidase is preferably 0.3 U or more, 0.6 U or more, 1 U or more, 1.3 U or more, 1.7 U or more, 2 U or more, 3 U or more, 4 U or more, 5 U or more, 7 U or more, 10 U or more, 30 U or more, 40 U or more, 70 U or more, 100 U or more, or 130 U or more. The upper limit of the range of the amount of the cellulase used per U of the protein deamidase is not particularly limited, and examples thereof include 600 U or less, 500 U or less, 400 U or less, 300 U or less, 200 U or less, 170 U or less, 150 U or less, 120 U or less, 90 U or less, 60 U or less, 40 U or less, 20 U or less, 9 U or less, 8 U or less, 6 U or less, 5 U or less, 4 U or less, 3 U or less, 2 U or less, 1.5 U or less, 0.8 U or less, or 0.4 U or less.

In the case of using, as the cellulase, a combination of a cellulase from Aspergillus and a cellulase from Trichoderma (preferably, a combination of a cellulase from Aspergillus niger and a cellulase from Trichoderma viride), examples of the amount of cellulase from Trichoderma used per U of cellulase from Aspergillus include 0.02 U or more, and from the viewpoint of further enhancing the effect of improving solubility, digestibility, and/or mineral solubility (overall solubility of calcium, iron, zinc and magnesium) of the protein, preferably 0.03 to 0.7 U, preferably 0.04 to 0.6 U, more preferably 0.045 to 0.3 U or 0.045 to 0.2 U, and still more preferably 0.05 to 0.1 U or 0.05 to 0.08 U.

It is to be noted that for the cellulase activity, the amount of enzyme that produces reducing sugar corresponding to 1 mg of glucose per minute is defined as 100 units (100 U) when the enzyme is treated at pH 4.5 and 40°C using carmellose sodium (sodium carboxymethylcellulose; degree of etherification: 0.62 to 0.68) as a substrate.

1-1-4. Reaction Conditions and the like

In the enzyme treatment step, the treatment proceeds a reaction that improves solubility, digestibility, and/or mineral solubility of the protein in the plant protein-containing liquid composition.

The order in which the protein deamidase and the cellulase are allowed to act is not particularly limited, and the respective enzymes may be allowed to act sequentially in any order, or both enzymes may be allowed to act simultaneously, but preferably both enzymes can act simultaneously.

The conditions (temperature, pH, time, etc.) of the enzyme treatment step are appropriately selected according to the characteristics of the enzyme and plant protein-containing liquid composition to be used, and the degree of the intended effect of improving solubility of the protein, digestibility, and/or mineral solubility.

The temperature at which the enzyme treatment step is performed is not particularly limited and can be appropriately determined by those skilled in the art according to the optimum temperature of the enzyme to be used and/or the thermal characteristics of the plant protein-containing liquid composition, and is, for example, 10°C to 70°C, preferably 25°C to 67°C, more preferably 30°C to 65°C, and still more preferably 45 to 63°C, 47 to 53°C, 55°C to 63°C, or 57 to 63°C.

The pH of the reaction system in which the enzyme treatment step is performed is not particularly limited and can be appropriately determined by those skilled in the art according to the optimum pH of the enzyme to be used and/or the pH characteristics of the plant protein-containing liquid composition, and the pH at 25°C is, for example, pH 1.0 to 8.0, preferably pH 3.0 to 8.0, more preferably pH 5.5 to 8.0, still more preferably pH 6.5 to 8.0, and even more preferably 7.0 to 7.5.

The time for performing the enzyme treatment step may be appropriately determined according to the scale of the plant protein-containing liquid composition to be subjected to the enzyme treatment step and/or the degree of the intended effect of improving solubility, digestibility, and/or mineral solubility of the protein, and examples thereof include 5 minutes to 48 hours, preferably 30 minutes to 20 hours, and more preferably 3 hours to 5 hours.

1-2. Other Steps

The method for producing a processed plant protein-containing liquid composition according to the present invention may or may not include any other steps than the protein deamidation step and the enzyme treatment step with a cellulase described in "1-1. Enzyme Treatment Step" as long as the effect of the present invention is obtained. Examples of the other steps include a step of preparing a plant protein-containing liquid composition, an amylase treatment step, a post-treatment step (cooling step, enzyme deactivation step, filtration step, etc.), and a drying step. These other steps may be performed singly or in combination with two or more steps. In addition, the timing of performing these other steps may not overlap or may overlap with the enzyme treatment step.

1-2-1. Step of Preparing Plant Protein-Containing Liquid Composition

The step of preparing the plant protein-containing liquid composition can be performed by any method capable of preparing the plant protein-containing liquid composition. Examples thereof include (I) a preparation method of dispersing a dry powder of a plant protein material in water, and if necessary, removing an insoluble matter derived from the peel or the like of the plant protein material by any means such as centrifugation, filtration, bag filtration, or sieving; (II) a preparation method including crushing and dispersing a plant protein material in water, and if necessary, removing an insoluble matter derived from the peel or the like of the plant protein material by any means such as centrifugation, filtration, bag filtration, or sieving; (III) a preparation method including removing at least one of components other than the plant protein from the liquid obtained by the method (I) or (II) to increase the content of a plant protein; and (IV) a preparation method of mixing a dry powder prepared from the liquid obtained by any one of the methods (I) to (III) with water.

1-2-2. Amylase Treatment Step

When the plant protein-containing liquid composition is prepared as an amylase-treated liquid composition, the liquid obtained by any one of the methods (I) to (IV) can be treated with amylase. The amylase treatment step may be performed before or simultaneously with the enzyme treatment step. Preferably, the amylase treatment step is performed before the enzyme treatment step. When the amylase treatment step is performed before the enzyme treatment step, the enzyme treatment step may be performed after the amylase is deactivated, or the enzyme treatment step may be performed without deactivating the amylase.

Examples of the amylase include α-amylase and β-amylase. Either one of these amylases may be used, or both may be used in combination, but α-amylase is preferably used.

The α-amylase is not particularly limited, and examples thereof include an α-amylase from the genus Aspergillus and an α-amylase from the genus Bacillus. More specifically, examples of the α-amylase from the genus Aspergillus include α-amylases from Aspergillus oryzae and Aspergillus niger, and examples of the α-amylase from the genus Bacillus include α-amylases from Bacillus amyloliquefaciens, Bacillus subtilis, Bacillus licheniformis, and Bacillus flexus. These α-amylases may be used singly or in combination with a plurality types thereof. Among these α-amylases, an α-amylase from the genus Bacillus is preferable, and an α-amylase from Bacillus amyloliquefaciens is more preferable.

The amount of α-amylase used is, for example, 0.001 U or more, preferably 0.05 U or more, more preferably 0.01 U or more, 0.05 U or more, 0.1 U or more, 0.2 U or more, or 0.5 U or more per g of starch contained in the plant protein-containing liquid composition. The upper limit of the range of the amount of α-amylase used is not particularly limited, and examples thereof include 100 U or less, 50 U or less, 10 U or less, 5 U or less, 1U or less, 0.6 U or less, 0.3 U or less, 0.1 U or less, 0.06 U or less, and 0.03 U or less.

For the α-amylase activity, the amount of enzyme that reduces the iodine-induced color development of potato starch by 10% per minute was defined as 1 unit (1 U).

The β-amylase is not particularly limited, and examples thereof include β-amylases derived from plants (wheat and soybean) and from the genus Bacillus, preferably β-amylases from the genus Bacillus, and more preferably β-amylases from the Bacillus flexus sp.

The amount of β-amylase used is, for example, 0.001 to 50 U, 0.01 to 10 U, 0.02 to 5.0 U, or 0.03 to 2.5U, preferably 0.05 to 1.5 U per g of starch contained in the plant protein-containing liquid composition.

For the β-amylase activity, the amount of enzyme that produces an increase in reducing power corresponding to 1 mg of glucose per minute is defined as 1 unit (1 U).

The conditions of the amylase treatment (treatment pH, treatment temperature, and treatment time) may be appropriately set according to the optimum pH and the optimum temperature of the enzyme used, the scale of the plant protein-containing liquid composition, and the like. Examples of the treatment pH of the oat protein-containing liquid composition before the amylase treatment include pH 5.5 to 8.0, preferably pH 6.5 to 8.0, and more preferably 7.0 to 7.5, in terms of pH at 25°C. The treatment temperature is, for example, 35°C to 70°C, preferably 40 to 67°C, and more preferably 45 to 65°C, 47 to 53°C, 55°C to 63°C, or 57 to 63°C. The treatment time is, for example, 10 minutes to 2 hours, preferably 20 minutes to 1 hour, and more preferably 20 to 40 minutes.

1-2-3. Enzyme Deactivation Step

In the enzyme deactivation step, the temperature condition and/or pH condition for deactivating the enzyme used can be appropriately selected in consideration of the temperature characteristics and/or pH characteristics of the plant protein-containing liquid composition, and preferably the temperature condition can be selected. Specific temperature conditions include, for example, 85 to 120°C, preferably 90 to 100°C, and more preferably 93 to 98°C, and the treatment time is, for example, 1 to 30 minutes, or 1 to 10 minutes, preferably 3 to 8 minutes.

1-2-4. Cooling Step, Filtration Step, and Drying Step

The cooling step, the filtration step, and/or the drying step can be performed, if necessary, after the completion of the enzyme deactivation step. In the drying step, the resulting processed plant protein-containing liquid composition can be prepared as a solid plant protein composition having improved solubility, digestibility, and/or mineral solubility of the protein. The drying method is not particularly limited, and examples thereof include freeze drying, vacuum drying, and spray drying. Examples of the shape of the solid plant protein composition include powder, fine particles, and granules.

2. Improver for Solubility, Digestibility, and/or Mineral Solubility of Protein in Plant Protein-containing Liquid Composition

The combination of a protein deamidase and a cellulase can improve the solubility, digestibility, and/or mineral solubility of the protein in the plant protein-containing liquid composition. Accordingly, the present invention also provides an improver for solubility, digestibility, and/or mineral solubility of a protein in a plant protein-containing liquid composition, the improver including a protein deamidase and a cellulase.

In the improver for solubility, digestibility, and/or mineral solubility of a protein in a plant protein-containing liquid composition, "improving the solubility, digestibility, and/or mineral solubility of the protein" means imparting, to the plant protein-containing liquid composition, at least one of a property of further increasing the amount of protein dissolved in water, a property of further increasing the amount of amino acid released in an internal environment, and a property of further increasing the amount of mineral (at least one of calcium, iron, zinc, and magnesium) dissolved in water, as compared with a case where the plant protein-containing liquid composition is treated with only a protein deamidase.

The improver for solubility, digestibility, and/or mineral solubility of the protein may be composed only of a protein deamidase and a cellulase, and on the other hand, may or may not further contain, as other components, an enzyme other than the protein deamidase and the cellulase, and/or a pharmaceutically acceptable additive and/or base of an enzyme agent. Examples of such an additive and base include an excipient, a buffer, an antioxidant, an ultraviolet inhibitor, a preservative, an antiseptic, a pH adjuster, a dispersant, an emulsifier, a solubilizing agent, a carrier, and a solvent (such as water). These additive and base may be used singly or in combination with two or more thereof. In addition, the contents of the additive and base may be appropriately set according to, for example, the type and/or formulation form of these components.

The properties of the improver for solubility, digestibility, and/or mineral solubility of the protein are not particularly limited, and examples thereof include dry preparations in the form of powder, fine particles, or granules, and liquid preparations.

In the improver for solubility, digestibility, and/or mineral solubility of the protein, the types and use ratios (blending ratios) of protein deamidase and cellulase as active ingredients, and the method of using the solubilizer are as described in the section of "1. Method for Producing Processed Plant Protein-containing Liquid Composition".

3. Improver for Solubility, Digestibility, and/or Mineral Solubility of Protein in Plant Protein-containing Liquid Composition Treated with Protein Deamidase

The cellulase can improve solubility, digestibility, and/or mineral solubility of a protein in a plant protein-containing liquid composition treated with a protein deamidase. Accordingly, the present invention also provides an improver for solubility, digestibility, and/or mineral solubility of a protein in a plant protein-containing liquid composition treated with a protein deamidase, the improver including a cellulase.

In the improver for solubility, digestibility, and/or mineral solubility of a protein in a plant protein-containing liquid composition treated with a protein deamidase, "improving the solubility, digestibility, and/or mineral solubility of the protein" means imparting, to the plant protein-containing liquid composition, at least one of a property of further increasing the amount of protein dissolved in water, a property of further increasing the amount of amino acid released in an internal environment, and a property of further increasing the amount of mineral (at least one of calcium, iron, zinc, and magnesium) dissolved in water, as compared with a case where the plant protein-containing liquid composition is treated with only a protein deamidase.

The improver for solubility, digestibility, and/or mineral solubility of the protein may be composed of only cellulase, and on the other hand, may or may not further contain, as other components, an enzyme other than the cellulase and protein deamidase, and/or a pharmaceutically acceptable additive and/or base of an enzyme agent. Examples of such an additive and base include an excipient, a buffer, an antioxidant, an ultraviolet inhibitor, a preservative, an antiseptic, a pH adjuster, a dispersant, an emulsifier, a solubilizing agent, a carrier, and a solvent (such as water). These additive and base may be used singly or in combination with two or more thereof. In addition, the contents of the additive and base may be appropriately set according to, for example, the type and/or formulation form of these components.

The properties of the improver for solubility, digestibility, and/or mineral solubility of the protein are not particularly limited, and examples thereof include dry preparations in the form of powder, fine particles, or granules, and liquid preparations.

In the improver for solubility, digestibility, and/or mineral solubility of the protein, the types of the cellulase as an active ingredient and the method of using the improver for solubility, digestibility, and/or mineral solubility of the protein are as described in the section of "1. Method for Producing Processed Plant Protein-containing Liquid Composition". A specific aspect of the method of using the improver for solubility, digestibility, and/or mineral solubility of the protein includes all of an aspect in which the plant protein-containing liquid composition is treated by simultaneously using the improver for solubility, digestibility, and/or mineral solubility of the protein and a protein deamidase, an aspect in which the plant protein-containing liquid composition is treated with the protein deamidase and then treated with the improver for solubility, digestibility, and/or mineral solubility of the protein, and an aspect in which the plant protein-containing liquid composition is treated with the improver for solubility, digestibility, and/or mineral solubility of the protein and then treated with the protein deamidase.

4. Plant Protein-containing Food and Drink Product

The plant protein-containing food and drink product of the present invention includes the processed plant protein-containing liquid composition obtained by "1. Method for Producing Processed Plant Protein-containing Liquid Composition" above. The processed plant protein-containing liquid composition is as described above in "1. Method for Producing Processed Plant Protein-containing Liquid Composition".

Specific forms of the plant protein-containing food and drink product of the present invention can be selected from any food and drink form.

Specific forms of the plant protein-containing food and drink product include plant alternative milk, alternative yogurt, alternative cheese, and alternative ice cream.

The plant protein-containing food and drink product can be obtained using the processed plant protein-containing liquid composition as it is or through an arbitrary preparing process. In such a preparing process, a preparing method according to the form of the plant protein-containing food and drink product is selected, and specifically, seasoning, form adjustment, molding, heating cooking, fermentation, freezing, and the like can be performed.

Examples

Hereinafter, the present invention will be described in detail with reference to Examples, but the present invention is not to be construed as being limited to the following Examples.

[Enzymes and Plant Protein Material Used]

In the following Examples, the following enzymes and plant protein materials were used.

[Table 1]

| Protein deamidase | From Chryseobacterium proteolyticum<br>Protein glutaminase | Amano Enzyme Inc. |
|---|---|---|
| α-Amylase | From Bacillus amyloliquefaciens<br>α-Amylase | Amano Enzyme Inc. |
| Cellulase 1 | From Aspergillus niger<br>Cellulase (mainly endoglucanase contained, with some exoglucanase mixed in. Endoglucanase activity >> Exoglucanase activity) | Amano Enzyme Inc. |
| Cellulase 2 | From Trichoderma viride<br>Cellulase (mainly exoglucanase contained, with some endoglucanase mixed in. Exoglucanase activity >> Endoglucanase activity) | Amano Enzyme Inc. |
| Protease | From Geobacillus stearothermophilus | Amano Enzyme Inc. |
| Oat flour | Premium oat flour<br>Protein content: 13.1 wt%<br>Carbohydrate content: 67.7 wt%<br>Dietary fiber content: 10.1 wt% | Slow Food |

[Enzyme Activity Measurement Method]

(1) Method of Measuring Protein Deamidase Activity

To 1 mL of 0.2 M phosphate buffer (pH 6.5) containing 30 mM Z-Gln-Gly, 0.1 mL of a sample solution containing a protein deamidase was added and allowed to stand at 37°C for 10 minutes, and then 1 mL of a 0.4 M TCA solution was added to stop the reaction. As a blank, 1 mL of a 0.4 M TCA solution was added to 1 mL of a 0.2M phosphate buffer (pH 6.5) containing 30 mM Z-Gln-Gly, 0.1 mL of a sample solution containing a protein deamidase was further added, and the mixture was allowed to stand at 37°C for 10 minutes.

For the solution obtained above, the amount of ammonia generated in the reaction solution was measured using Ammonia Test Wako (FUJIFILM Wako Pure Chemical Corporation). The ammonia concentration in the reaction solution was determined from a calibration curve representing the relationship between the ammonia concentration and the absorbance (630 nm) prepared using an ammonia standard solution (ammonium chloride).

The protein deamidase activity was calculated according to the following formula, where the amount of enzyme producing 1 μmol of ammonia per minute was defined as 1 unit (1 U). In the formula, the reaction solution volume is 2.1, the enzyme solution volume is 0.1, and Df is the dilution factor of the enzyme solution. In addition, 17.03 is the molecular weight

of ammonia.

[Formula 1]

Protein deamidase activity (U/mL)

= ammonia concentration in reaction solution (mg/L) × (1/17.03) ×

(reaction solution volume/enzyme solution volume) × (1/10) × Df

(2) Cellulase Activity Measurement Method

About 1 g of carmellose sodium (sodium carboxymethylcellulose; degree of etherification: 0.62 to 0.68) was precisely weighed and dried at 105°C for 4 hours to measure the weight loss. Carmellose sodium corresponding to 0.500 g of the dried product was weighed, and slowly (about 30 minutes) was added to and dissolved in hot water at 60 to 70°C in about 50 mL of continuous stirring with a stirrer. Then, 10 mL of a 1 mol/L acetic acid-sodium acetate buffer solution (pH 4.5) and water were added to make 100 mL, which was used as a substrate solution.

In a 50 mL Nessler tube, 4 mL of a substrate solution (pH 4.5) was weighed and allowed to stand at 40°C for 10 to 15 minutes. Then, 1 mL of a sample solution containing a cellulase was added thereto, and the mixture was immediately shaken. After this liquid was allowed to stand at 40°C for exactly 30 minutes, 2 mL of somogyi reagent 1 was added, and the mixture was shaken, capped, heated in a boiling water bath for exactly 20 minutes, and then immediately cooled. After cooling, 1 mL of the Nelson's reagent was added, and the mixture was shaken well until a red precipitate of cuprous oxide was completely dissolved and allowed to stand at room temperature for about 20 minutes. Subsequently, 17 mL of water was added to make a total volume of 25 mL, and the mixture was shaken well. The absorbance (A30) of the resulting liquid at a wavelength of 500 nm was measured using water as a control. Separately, 2 mL of Somogyi reagent 1 was added to 4 mL of the substrate solution (pH 4.5), and the mixture was shaken. Then, 1 mL of a sample solution containing a cellulase was added thereto, and the mixture was operated in the same manner as described above to measure the absorbance (A0).

The cellulase activity was calculated according to the following formula, where the amount of enzyme producing reducing sugar corresponding to 1 mg of glucose per minute was defined as 100 units (100 U).

[Formula 2]

Cellulase activity per g or mL of sample (U/g, U/mL) = G × 1/30 × 100 × n

G: Generated glucose content (mg) determined from glucose calibration curve based on (A30-A0) value
1/30: Conversion factor per minute
100: Conversion factor to 100 units
n: Dilution factor per g or mL of sample
a: Slope of glucose calibration curve
b: Intercept of glucose calibration curve

(3) Method of Measuring α-Amylase Activity

After 10 mL of a 1% potato starch substrate solution (0.1 mol/L acetic acid (pH 5.0)) was heated at 37°C for 10 minutes, 1 mL of a sample solution containing α-amylase was added, and the mixture was immediately shaken. This liquid was allowed to stand at 37°C for 10 minutes, then 1 mL of the liquid was added to 10 mL of a 0.1 mol/L hydrochloric acid solution, and the mixture was immediately shaken. Next, 0.5 mL of this liquid was weighed, 10 mL of a 0.0002 mol/L iodine solution (JP) was added thereto, and the mixture was shaken, and then the absorbance (AT) at a wavelength of 660 nm was measured using water as a control. Separately, 1 mL of water was added instead of the sample solution, and the same procedure was carried out to measure the absorbance (AB). The amount of enzyme that reduced the iodine-induced color development of potato starch by 10% per minute was defined as 1 unit (1 U).

[Formula 3]

$$\alpha\text{-Amylase activity (U/g, U/mL)} = (AB - AT)/AB \times 1/W$$

AT: Absorbance of reaction solution
AB: Absorbance of blank liquid
W: Amount of sample in 1 mL of sample solution (g or mL)

(4) Protease Activity Measurement Method

After 5 mL of a 0.6% (w/v) casein solution (0.05 mol/L sodium hydrogen phosphate, pH 8.0) was heated at 37°C for 10 minutes, 1 mL of a sample solution containing protease was added, and the mixture was immediately shaken. This liquid was allowed to stand at 37°C for 10 minutes, 5 mL of a trichloroacetic acid test solution (1.8% (w/v) trichloroacetic acid, 1.8% (w/v) sodium acetate, and 0.33 mol/L acetic acid) was added. Then, the mixture was shaken, allowed to stand again at 37°C for 30 minutes, and filtered. The first filtrate (3 mL) was removed, and the next filtrate (2 mL) was weighed. Then, 5 mL of a 0.55 mol/L sodium carbonate test solution and 1 mL of a Folin's reagent (1→3) were added, and the mixture was shaken well and allowed to stand at 37°C for 30 minutes. The absorbance AT of the liquid (enzyme reaction solution) at a wavelength of 660 nm was measured using water as a control.

The absorbance AB of a liquid (blank) that was operated in the same manner as the above-described enzyme reaction solution was measured except that, separately, 1 mL of a sample solution containing protease was weighed, and 5 mL of a trichloroacetic acid test solution (1.8% (w/v) trichloroacetic acid, 1.8% (w/v) sodium acetate, and 0.33 mol/L acetic acid) was added and shaken, then 5 mL of a 0.6% (w/v) casein solution (0.05 mol/L sodium hydrogen phosphate, pH 8.0) was added, and the mixture was immediately shaken and allowed to stand at 37°C for 30 minutes.

The amount of enzyme that produced an increase in the amount of the Folin's reagent coloring substance corresponding to 1 $\mu$g of tyrosine per minute was defined as 1 unit (1 U).

Each of 1 mL, 2 mL, 3 mL, and 4 mL of 1 mg/mL tyrosine standard stock solution (0.2 mol/L hydrochloric acid) was weighed, and a 0.2 mol/L hydrochloric acid test solution was added thereto to make 100 mL. To 2 mL of each solution, 5 mL of a 0.55 mol/L sodium carbonate test solution and 1 mL of a Folin's reagent (1→3) were added, and the mixture was immediately shaken and allowed to stand at 37°C for 30 minutes. For these liquids, absorbances A1, A2, A3 and A4 at a wavelength of 660 nm were measured using, as a control, a liquid obtained by weighing 2 mL of a 0.2 mol/L hydrochloric acid test solution and operating in the same manner as described above. The absorbances A1, A2, A3 and A4 were plotted on the vertical axis, and the amount of tyrosine ($\mu$g) in 2 mL of each liquid was plotted on the horizontal axis. A calibration curve was prepared to determine the amount of tyrosine ($\mu$g) with respect to the absorbance difference 1.

[Formula 4]

$$\text{Protease activity (U/g, U/mL)} = (AT-AB) \times F \times 11/2 \times 1/10 \times 1/M$$

AT: Absorbance of enzyme reaction solution
AB: Absorbance of blank
F: Amount of tyrosine ($\mu$g) when absorbance difference determined from tyrosine calibration curve is 1
11/2: Conversion factor to total liquid volume after reaction is stopped
1/10: Conversion factor to reaction time per minute
M: Amount of sample in 1 mL of sample solution (g or mL)

[Test Example 1]

(1) Production of Processed Oat Milk

First, 18.1 g of oat flour and 150 g of water were mixed and suspended in a 300 mL conical flask. To this suspension (oat flower content: 10.8 wt%, protein content: 1.4 wt%, dietary fiber content: 1.1 wt%, starch content: 7.3 wt%), 0.7 U/g-starch $\alpha$-amylase was added and reacted at 60°C for 0.5 hours to prepare oat milk (pH 7.0 to 7.5 at 25°C). Thereafter, 10 U/g-protein protein deamidase and 1500 U/g-dietary fiber cellulase 1 were added thereto, and the mixture was reacted at 60°C for 2 hours or 4 hours. After the reaction, the enzyme was deactivated by heat treatment at 95°C for 5 minutes. Thus, processed oat milk (Examples 1 and 2) was obtained. In addition, the same operation was performed except that cellulase 1 was not added to obtain processed oat milk for comparison (Comparative Examples 1 and 2). The obtained processed oat milk was subjected to the following test.

(2) Testing of Processed Oat Milk

(2-1) Protein Solubility Measurement

· Method for Preparing Sample Liquid for Measurement of Processed Oat Milk

The processed oat milk was centrifuged at 9,000 rpm for 10 minutes, and then the supernatant was recovered. The supernatant was further diluted 50-fold to prepare a sample liquid for measurement.

· Method for Preparing BCA Solution

A BCA solution was prepared by mixing 50 mL of BCA Protein Assay Reagent (Reagent A Pierce) and 1 mL of BCA Protein Assay Reagent (Reagent B Pierce) well.

· Protein Solubility Measurement Method

First, 4 mL of the BCA solution was weighed and allowed to stand at $37 \pm 0.5°C$ for exactly 10 minutes. Then, 0.2 mL of the sample liquid for measurement was added, and the mixture was immediately shaken. This liquid was allowed to stand at $37 \pm 0.5°C$ for exactly 30 minutes and then cooled in running water (20 to 25°C). The absorbance (AT) of this liquid at a wavelength of 562 nm was measured using water as a control. The absorbance (AB) of 4 mL of the BCA solution, which was allowed to stand at $37 \pm 0.5°C$ for exactly 10 minute and the shaken with 0.2 mL of water, was measured by the same operation as described above.

The albumin concentration Tmg in the sample diluted solution was determined from the absorbance difference (AT-AB) and the albumin standard curve. The protein solubility (mg/g) in the processed oat milk was determined based on the obtained albumin concentration Tmg. The protein solubility in the processed oat milk of Examples 1 and 2 was derived as the relative solubility (%) when the protein solubility in the processed oat milk of Comparative Examples 1 and 2 was 100%. The results are shown in Table 2.

(2-2) Results

[Table 2]

| | PG addition activity (U/g-protein) | Cellulase 1 addition activity (U/g-dietary fiber) | Treatment condition | Protein relative solubility (%) |
|---|---|---|---|---|
| Comparative Example 1 | 10 | - | 60°C, 2 h | 100 |
| **Example 1** | **10** | **1500** | **60°C, 2 h** | **124.5** |
| Comparative Example 2 | 10 | - | 60°C, 4 h | 100 |
| **Example 2** | **10** | **1500** | **60°C, 4 h** | **131.2** |

As shown in Table 2, the protein solubility was improved in the processed oat milk (Examples 1 and 2) obtained by a combined treatment of protein glutaminase (PG) and cellulase 1, as compared with the processed oat milk (Comparative Examples 1 and 2) obtained by a single treatment of protein glutaminase (PG).

In the processed oat milk (Examples 3 and 4) produced in the same manner as in Examples 1 and 2 except that cellulase 2 (cellulase from Trichoderma viride) was used instead of cellulase 1 (cellulase from Aspergillus niger), it was confirmed that the protein solubility was also improved in the same manner as in Examples 1 and 2.

Furthermore, in the processed oat milk (Examples 1a and 2a) produced by changing the amount of cellulase 1 added to 74.6 U/g-dietary fiber in Examples 1 and 2 and the processed oat milks (Examples 3a and 4a) produced by changing the amount of cellulase 2 added to 14 U/g-dietary fiber in Examples 3 and 4, it was confirmed that the protein solubility in the produced processed oat milk was also improved in the same manner as in Examples 1 and 2 and Examples 3 and 4.

[Test Example 2]

(1) Production of Processed Oat Milk

In "(1) Production of Processed Oat Milk" of Test Example 1, the enzyme to be added to the oat milk was as shown in Table 3, and the enzyme reaction time was set to 4 hours to prepare processed oat milk (Comparative Example 2, Comparative Example 4, and Example 2). Comparative Example 3 was obtained by performing the same operation except that no enzyme was added.

(2) Testing of Processed Oat Milk

(2-1) Protein Solubility Measurement

The processed oat milk was centrifuged at 9,000 rpm for 5 minutes, and then the supernatant and the residue were recovered. The residue was dried at 80°C for 6 hours. The supernatant and the dry residue were each used as a measurement sample.
The protein content contained in each of the measurement samples (supernatant and dry residue) was measured as a nitrogen content by the Kjeldahl method. The results are shown in Table 3.

(2-2) Results

[Table 3]

|  | PG addition activity (U/g-protein) | Cellulase 1 addition activity (U/g-dietary fiber) | Treatment condition | Protein content in dry residue (%) | **Protein content in supernatant (%)** |
|---|---|---|---|---|---|
| Comparative Example 3 | - | - | 60°C, 4 h | 6.76 | **1.06** |
| Comparative Example 2 | 10 | - | 60°C, 4 h | 2.42 | **5.82** |
| Comparative Example 4 | - | 1500 | 60°C, 4 h | 6.68 | **1.10** |
| **Example 2** | **10** | **1500** | **60°C, 4 h** | **1.90** | **6.94** |

As shown in Table 3, comparing the protein content of the enzyme-untreated (Comparative Example 3) with the protein content of the enzyme-treated (Comparative Examples 2 and 4 and Example 2), it was confirmed that the protein solubility was improved in the processed oat milk obtained by the combined treatment of PG and cellulase 1 (Example 2) since the protein in the residue decreased and the protein in the supernatant increased synergistically, as compared with the processed oat milk obtained by the single treatment of protein glutaminase (PG) (Comparative Example 2) and the processed oat milk obtained by the single treatment of cellulase 1 (Comparative Example 4).
In Comparative Example 4 and Example 2, the same operation was also performed on the processed oat milk (Comparative Example 4a and Example 2a) produced by changing the amount of cellulase 1 added to 74.6 U/g-dietary fiber. As a result, comparing the protein content of the enzyme-untreated (Comparative Example 3) with the protein content of the enzyme-treated (Comparative Examples 2 and 4a and Example 2a), it was confirmed that, as in Table 3, the protein solubility was improved in the processed oat milk obtained by the combined treatment of PG and cellulase 1 (Example 2a) since the protein in the residue decreased and the protein in the supernatant increased synergistically, as compared with the processed oat milk obtained by the single treatment of protein glutaminase (PG) (Comparative Example 2) and the processed oat milk obtained by the single treatment of cellulase 1 (Comparative Example 4a).

[Test Example 3]

(1) Production of Processed Oat Milk

In "(1) Production of Processed Oat Milk" of Test Example 1, the addition amount of $\alpha$-amylase used in the production of oat milk to 0.01 U/g-starch, the treatment temperature with $\alpha$-amylase was set to 60°C, the enzymes to be added to the obtained oat milk were as shown in Tables 4 to 6, and the enzyme treatment temperature was adjusted to 50°C to prepare processed oat milk (Comparative Examples 6 and 7 and 9 to 13, and Examples 5 to 9). Comparative Examples 5 and 8 were obtained by performing the same operation except that no enzyme was added.

(2) Testing of Processed Oat Milk

(2-1) Protein Solubility Measurement

The measurement was performed in the same manner as in "(2-1) Protein Solubility Measurement" of Test Example 2. The results are shown in Tables 4 to 6.

(2-2) Results

[Table 4]

| | PG addition activity (U/g-protein) | Cellulase addition activity (U/g-dietary fiber) | | Treatment condition | Protein content (%) | |
|---|---|---|---|---|---|---|
| | | Cellulase 1. | Cellulase 2 | | Residue | Supernatant |
| Comparative Example 5 | - | - | - | 50°C, 2 h | 31.6 | 0.09 |
| Comparative Example 6 | 10 | - | - | 50°C, 2 h | 20.8 | 0.51 |
| Comparative Example 7 | - | - | 14 | 50°C, 2 h | 28.5 | 0.22 |
| **Example 5** | **10** | **-** | **14** | **50°C,2 h** | **18.4** | **0.67** |
| Comparative Example 8 | - | - | - | 50°C, 4 h | 31.4 | 0.11 |
| Comparative Example 9 | 10 | - | - | 50°C, 4 h | 20.1 | 0.55 |
| Comparative Example 10 | - | - | 14 | 50°C, 4 h | 28.9 | 0.23 |
| **Example 6** | **10** | **-** | **14** | **50°C, 4 h** | **16.5** | **0.85** |

[Table 5]

| | PG addition activity (U/g-protein) | Cellulase addition activity (U/g-dietary fiber) | | Protease addition activity (U/g-protein) | Treatment condition | Protein content (%) |
|---|---|---|---|---|---|---|
| | | Cellulase 1 | Cellulase 2 | | | Supernatant |
| Comparative Example 8 | - | - | - | - | 50°C, 4 h | 0.11 |
| Comparative Example 9 | 10 | - | - | - | 50°C, 4 h | 0.55 |
| Comparative Example 11 | - | 74.6 | - | - | 50°C, 4 h | 0.10 |
| **Example 7** | **10** | **74.6** | **-** | **-** | **50°C, 4 h** | **0.72** |
| Comparative Example 10 | - | - | 14 | - | 50°C, 4 h | 0.23 |
| Example 6 | **10** | **-** | **14** | **-** | **50°C, 4 h** | **0.85** |
| **Example 8** | **10** | **74.6** | **14** | **-** | **50°C, 4 h** | **1.11** |

[Table 6]

| | PG addition activity (U/g-protein) | Cellulase addition activity (U/g-dietary fiber) | | Protease addition activity (U/g-protein) | Treatment condition | Protein content (%) |
| --- | --- | --- | --- | --- | --- | --- |
| | | Cellulase 1 | Cellulase 2 | | | Supernatant |
| Comparative Example 8 | - | - | - | - | 50°C, 4 h | 0.11 |
| Comparative Example 9 | 10 | - | - | - | 50°C, 4 h | 0.55 |
| Comparative Example 11 | - | 74.6 | - | - | 50°C, 4 h | 0.10 |
| Comparative Example 12 | - | - | - | 100 | 50°C, 4 h | 0.16 |
| Comparative Example 13 | 10 | - | - | 100 | 50°C, 4 h | 0.75 |
| **Example 9** | **10** | **74.6** | **-** | **100** | **50°C, 4 h** | **1.03** |

As shown in Table 4, comparing the protein content of the enzyme-untreated (Comparative Examples 5 and 8) with the protein content of the enzyme-treated (Comparative Examples 6 and 7, Example 5, Comparative Examples 9 and 10, and Example 6), it was confirmed that the protein solubility was improved in the processed oat milk obtained by the combined treatment of PG and cellulase 2 (Examples 5 and 6) since the protein in the residue decreased and the protein in the supernatant increased synergistically, as compared with the processed oat milk obtained by the single treatment of protein glutaminase (PG) (Comparative Examples 6 and 9) and the processed oat milk obtained by the single treatment of cellulase 2 (Comparative Examples 7 and 10).

As shown in Table 5, when the protein content of the enzyme-untreated (Comparative Example 8) was compared with the protein content of the enzyme-treated (Comparative Examples 9 to 11 and Examples 6 to 8), it was confirmed that the protein in the supernatant was synergistically increased in the processed oat milk (Examples 7 and 6) obtained by the combined treatment of PG and each cellulase 1 or 2, compared with the processed oat milk obtained by the single treatment of protein glutaminase (PG) (Comparative Example 9) and the processed oat milk obtained by the single treatment of each cellulase 1 or 2 (Comparative Examples 11 and 10), and the protein solubility was further improved in the processed oat milk (Example 8) obtained by the combined treatment of PG, cellulase 1, and cellulase 2, because the protein in the supernatant was further synergistically increased.

As shown in Table 6, comparing the protein content of the enzyme-untreated (Comparative Example 8) with the protein content of the enzyme-treated (Comparative Examples 9 to 13 and Example 9), it was confirmed that the protein solubility was improved in the processed oat milk obtained by the combined treatment of PG, cellulase 1, and protease (Example 9), because the protein in the supernatant increased synergistically, compared with the processed oat milk obtained by the single treatment of protein glutaminase (PG) (Comparative Example 9), the processed oat milk obtained by the single treatment of cellulase 1 (Comparative Example 11), and the processed oat milk obtained by the single treatment of protease (Comparative Example 12).

[Test Example 4]

(1) Production of Processed Oat Milk

In "(1) Production of Processed Oat Milk" of Test Example 1, the enzyme to be added to the oat milk was as shown in Table 7, and the enzyme reaction time was set to 4 hours to prepare processed oat milk (Comparative Example 2, Comparative Example 4, Comparative Example 14, Example 2, and Example 4). Comparative Example 3 was obtained by performing the same operation except that no enzyme was added.

(2) Testing of Processed Oat Milk

(2-1) Digestibility Test

An in vitro digestion test was performed using 5 g of the prepared processed oat milk. The in vitro digestion test referred to the INFOGEST method (Brodkorb et al. 2019 Nat. Protoc).

First, 3.5 ml of artificial saliva and 0.5 ml of 0.77 mg/mL amylase were mixed with 5 g of processed oat milk, and the mixture was stirred at 160 rpm in a water bath at 37°C for 2 minutes to obtain an oral digest model. The composition of the artificial saliva was 0.28 M KCl, 0.07 mM $KH_2PO_4$, 0.26 mM $NaHCO_3$, 2.8 mM $MgCl_2$, 1.1 mM $(NH_4)_2CO_3$, and 1.5 mM

CaCl$_2$(pH 7.0).

Next, 7.5 ml of artificial gastric fluid and 1.6 ml of a 0.227 g/19.2 mL pepsin solution (Sigma-Aldrich) were mixed with 10 ml of the oral digest model, and the mixture was stirred at 160 rpm in a water bath at 37°C for 2 hours to obtain a gastric digest model. The composition of the artificial gastric fluid was 0.10 M KCl, 0.01 M KH$_2$PO$_4$, 0.38 M NaHCO$_3$, 0.71 M NaCl, 1.82 mM MgCl$_2$, 7.58 mM (NH$_4$)$_2$CO$_3$, 0.165 mM CaCl$_2$ (pH 3.0).

Furthermore, 11 ml of artificial intestinal fluid, 16.4 mg/5 mL of a pancreatin solution (Sigma-Aldrich), 215 mg/2.5 mL of sodium taurocholate, and 40 μL of a 0.3M calcium chloride solution were mixed with 20 ml of the gastric digest model, and the mixture was stirred at 160 rpm in a water bath at 37°C for 2 hours to obtain a small intestine digest model. The composition of the artificial intestinal fluid was 8.5 mM KCl, 1 mM KH$_2$PO$_4$, 106.3 mM NaHCO$_3$, 48 mM NaCl, and 0.4 mM MgCl$_2$ (pH 7.0).

[Measurement of Free Amino Nitrogen Content]

To 1.0 mL of a diluted solution obtained by appropriately diluting a supernatant of the oral digest model, the gastric digest model, or the small intestine digest model, 0.5 mL of a ninhydrin reagent with pH 6.7 (10% Na$_2$HPO$_4$/12H$_2$O, 6% KH$_2$PO$_4$, 0.50% ninhydrin, and 0.30% fructose) was added and mixed. Thereafter, the mixture was boiled for 16 minutes, and the content (mg/L) of free amino nitrogen was measured at an absorbance of 570 nm. The higher the content of free amino nitrogen is, the more amino acids are liberated from the protein when digested, that is, the higher the digestibility of the protein is. The results are shown in Table 7.

[Amino Acid Analysis]

The supernatant of the small intestine digest model was filtered through a filter (0.45 μm) to prepare an amino acid analysis sample. Separately, a supernatant of the small intestinal digest model was prepared by performing the above digestibility test, except that PG and/or cellulase were not added, and further filtered through a filter (0.45 μm) to prepare a control amino acid analysis sample. Each of the amino acid analysis sample and the control amino acid analysis sample was analyzed for the amount of free amino acids using an amino acid analyzer (amino acid analysis using Agilent 1260 Infinity II LC system) according to the protocol of the analyzer. Specifically, first, free essential amino acids (EAAs) [specifically, branched chain amino acids ((BCAAs); valine, isoleucine, and leucine), aromatic amino acids ((AAAs); phenylalanine and tryptophan), and other essential amino acids (methionine, lysine, histidine, arginine, and threonine)] and non-essential amino acids [(NEAAs); glycine, alanine, serine, tyrosine, cysteine, asparagine, glutamine, proline, aspartic acid, and glutamic acid] were measured. The ratio (EAA/NEAA) of the free amount of EAA (total free amount of BCAA, AAA, and other EAAs) to the free amount of NEAA in amino acid analysis samples was derived. When the ratio EAA/NEAA in Comparative Example 3 was 1, the relative amount of the ratio EAA/NEAA in each of Examples and Comparative Examples was derived. If the amount exceeds 1, it means that the absorption amount of EAA is increased by digestion as compared with NEAA. The results are shown in Table 7.

(2-2) Results

[Table 7]

| | PG addition activity (U/g-protein) | Cellulase addition activity (U/g-dietary fiber) | | Heating condition | Free amino nitrogen content (mg/L) | | | Ratio (EAA/NEAA) |
| | | Cellulase 1 | Cellulase 2 | | Oral cavity | Stomach | Small intestine | |
|---|---|---|---|---|---|---|---|---|
| Comparative Example 3 | - | - | - | 60°C, 4 h | 0.0 | 14.2 | 28.1 | 1.00 |
| Comparative Example 2 | 10 | - | - | 60°C, 4 h | 0.0 | 18.8 | 44.7 | 1.21 |
| Comparative Example 4 | - | 1500 | - | 60°C, 4 h | 0.0 | 14.5 | 28.3 | 1.09 |
| Comparative Example 14 | - | - | 1500 | 60°C, 4 h | 0.0 | 16.1 | 33 | 1.11 |

(continued)

|  | PG addition activity (U/g-protein) | Cellulase addition activity (U/g-dietary fiber) | | Heating condition | Free amino nitrogen content (mg/L) | | | Ratio (EAA/NEAA) |
|---|---|---|---|---|---|---|---|---|
|  |  | Cellulase 1 | Cellulase 2 |  | Oral cavity | Stomach | Small intestine |  |
| Example 2 | 10 | 1500 | - | 60°C, 4 h | 0.0 | 19.1 | 45.4 | 1.24 |
| Example 4 Example | 10 | - | 1500 | 60°C, 4 h | 0.0 | 24.1 | 52.3 | 1.35 |

As shown in Table 7, compared with the processed oat milk obtained by the single treatment with protein glutaminase (PG) (Comparative Example 2), the processed oat milk obtained by the combined treatment with PG and either cellulase 1 or 2 (Examples 2 and 4) showed improved protein digestibility and improved liberation efficiency of essential amino acids by digestion. In particular, in the processed oat milk (Example 4) obtained by the combined treatment of PG and cellulase 2, the effect of improving digestibility of protein and the efficiency of liberation of essential amino acids by digestion were further improved.

Further, the same operation was performed on the processed oat milk (Comparative Example 4a and Example 2a) produced by changing the amount of cellulase 1 added to 74.6 U/g-dietary fiber in Comparative Example 4 and Example 2, and the processed oat milk (Comparative Example 14a and Example 4a) produced by changing the amount of cellulase 2 added to 14 U/g-dietary fiber in Comparative Example 14 and Example 4. As a result, as in Table 7, compared with the processed oat milk obtained by the single treatment with protein glutaminase (PG) (Comparative Example 2), the processed oat milk obtained by the combined treatment with PG and either cellulase 1 or 2 (Examples 2a and 4a) showed improved protein digestibility and improved liberation efficiency of essential amino acids by digestion. In particular, in the processed oat milk (Example 4a) obtained by the combined treatment of PG and cellulase 2, the effect of improving digestibility of protein and the efficiency of liberation of essential amino acids by digestion were further improved.

[Test Example 5]

(1) Production of Processed Oat Milk

In "(1) Production of Processed Oat Milk" of Test Example 1, the addition amount of $\alpha$-amylase used in the production of oat milk to 0.01 U/g-starch, the treatment temperature with $\alpha$-amylase was set to 60°C, the enzyme to be added to oat milk was as shown in Table 9, and the enzyme treatment conditions was adjusted to 50°C and 4 hours to prepare processed oat milk (Comparative Examples 9 to 11 and Examples 6, 7, and 10 to 12).

(2) Testing of Processed Oat Milk

(2-1) Measurement of Amount of Minerals

The obtained processed oat milk was subjected to centrifugation (15,000 rpm, 5 minutes) to obtain a supernatant. The amounts of calcium, iron, zinc, and magnesium in the obtained supernatant were measured using the following mineral measurement kit.

[Table 8]

| Calcium | Metallo Assay "calcium measurement LS (CPZIII)" | Manufactured by Metallogenics Co., Ltd. |
|---|---|---|
| Iron | Metallo Assay "iron measurement LS (ferrozine method)" | Manufactured by Metallogenics Co., Ltd. |
| Zinc | Metallo Assay "zinc measurement LS" | Manufactured by Metallogenics Co., Ltd. |
| Magnesium | Metallo Assay "magnesium measurement LS" | Manufactured by Metallogenics Co., Ltd. |

(2-2) Results

[Table 9]

| | PG addition activity (U/g-protein) | Cellulase addition activity (U/g-dietary fiber) | | Amount of soluble minerals (g/L) | | | |
|---|---|---|---|---|---|---|---|
| | | Cellulase 1 | Cellulase 2 | Ca | Fe | Zn | Mg |
| Comparative Example 9 | 10 | - | - | $1.3 \times 10^{-5}$ | $6.7 \times 10^{-2}$ | $38.9 \times 10^{-5}$ | $3.7 \times 10^{-2}$ |
| Comparative Example 11 | - | 74.6 | - | no data | no data | $3.3 \times 10^{-5}$ | $0.56 \times 10^{-2}$ |
| Comparative Example 10 | - | - | 14 | no data | no data | $0.37 \times 10^{-5}$ | $0.05 \times 10^{-2}$ |
| **Example 7** | **10** | **74.6** | **-** | **$1.8 \times 10^{-5}$** | **$12.6 \times 10^{-2}$** | **$56.2 \times 10^{-5}$** | **$5.0 \times 10^{-2}$** |
| **Example 10** | **10** | **56.0** | **3** | **$2.2 \times 10^{-5}$** | **$14.3 \times 10^{-2}$** | **$55.0 \times 10^{-5}$** | **$5.0 \times 10^{-2}$** |
| **Example 11** | **10** | **37.3** | **7** | **$2.0 \times 10^{-5}$** | **$13.7 \times 10^{-2}$** | **$51.6 \times 10^{-5}$** | **$4.8 \times 10^{-2}$** |
| **Example 12** | **10** | **18.7** | **10** | **$1.7 \times 10^{-5}$** | **$12.9 \times 10^{-2}$** | **$50.7 \times 10^{-5}$** | **$4.5 \times 10^{-2}$** |
| **Example 6** | **10** | **-** | **14** | **$1.6 \times 10^{-5}$** | **$8.8 \times 10^{-2}$** | **$48.2 \times 10^{-5}$** | **$4.4 \times 10^{-2}$** |

In all Comparative Examples and Examples in the table, the treatment conditions are 50°C and 4 hours.

As shown in Table 9, the amount of soluble minerals including calcium was improved in the processed oat milk (Examples 7, 10 to 12, and 6) obtained by the combined treatment of protein glutaminase (PG) with cellulase 1 and/or cellulase 2, as compared with the processed oat milk obtained by the single treatment of protein glutaminase (PG) (Comparative Example 9). Although the amount of soluble minerals was extremely small in the single treatment of cellulases 1 and 2, the amount of soluble minerals was remarkably improved by combining with PG. With respect to zinc and magnesium, from the comparison between the effect of improving the amount of soluble minerals by the combined treatment of PG and cellulase 1 (Example 7) with respect to the single treatment of cellulase 1 (Comparative Example 11) and the effect of improving the amount of soluble minerals by the combined treatment of PG and cellulase 2 (Example 6) with respect to the single treatment of cellulase 2 (Comparative Example 10), the effect of improving the amount of soluble minerals by the combination with PG (effect per activity) was superior in cellulase 2. Calcium and iron were further remarkably improved by combining both cellulase 1 and cellulase 2 at a predetermined ratio. When all the minerals were comprehensively evaluated, the effect of improving the amount of soluble minerals of Example 10 was the best.

**Claims**

1. A method for producing a processed plant protein-containing liquid composition, the method comprising treating a plant protein-containing liquid composition with a protein deamidase and cellulase.

2. The method for producing a processed plant protein-containing liquid composition according to claim 1, wherein the plant protein is a cereal protein.

3. The method for producing a processed plant protein-containing liquid composition according to claim 1, wherein the plant protein is an oat protein.

4. The method according to claim 1, wherein the cellulase is a cellulase from the genus Aspergillus and/or cellulase from the genus Trichoderma.

5. The method according to claim 1, wherein in the cellulase, exoglucanase activity is higher than endoglucanase activity.

6. The method for producing a processed plant protein-containing liquid composition according to claim 1, wherein the cellulase is used in an amount of 2 to 5000 U per U of the protein deamidase.

7. The method for producing a processed plant protein-containing liquid composition according to claim 1, wherein the protein deamidase is used in an amount of 0.01 to 200 U per g of the plant protein.

8. A method for improving solubility, digestibility, and/or mineral solubility of a protein in a plant protein-containing liquid composition, the method comprising treating the plant protein-containing liquid composition with a protein deamidase and cellulase.

9. An improver for solubility, digestibility, and/or mineral solubility of a protein in a plant protein-containing liquid composition, the improver comprising a protein deamidase and a cellulase.

10. The improver for solubility, digestibility, and/or mineral solubility of a protein according to claim 9, the improver comprising 2 to 5000 U of the cellulase per U of the protein deamidase.

11. An improver for solubility, digestibility, and/or mineral solubility of a protein in a plant protein-containing liquid composition treated with a protein deamidase, the improver comprising a cellulase.

12. A plant protein-containing food and drink product comprising a processed plant protein-containing liquid composition obtained by the method according to claim 1.

## INTERNATIONAL SEARCH REPORT

| International application No. |
|---|
| **PCT/JP2024/005385** |

**A.  CLASSIFICATION OF SUBJECT MATTER**

*A23L 33/185*(2016.01)i; *C07K 1/12*(2006.01)i; *C12P 21/00*(2006.01)i
FI:   A23L33/185; C07K1/12; C12P21/00 A

According to International Patent Classification (IPC) or to both national classification and IPC

**B.  FIELDS SEARCHED**

Minimum documentation searched (classification system followed by classification symbols)

A23L33/185; C07K1/12; C12P21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580/JSTChina (JDreamIII), Google

**C.  DOCUMENTS CONSIDERED TO BE RELEVANT**

| Category* | Citation of document, with indication, where appropriate, of the relevant passages | Relevant to claim No. |
|---|---|---|
| X | WO 2022/063833 A1 (SOCIETE DES PRODUITS NESTLE S.A.) 31 March 2022 (2022-03-31)<br>claims, page 8, lines 11-14, example 7 | 1, 2, 4-12 |
| A | | 3 |
| X | CN 115669852 A (HEBEI UNIVERSITY OF SCIENCE AND TECHNOLOGY) 03 February 2023 (2023-02-03)<br>claims, paragraphs [0017], [0018], examples | 1-12 |
| A | WO 2022/138872 A1 (AMANO ENZYME INC.) 30 June 2022 (2022-06-30)<br>entire text, all drawings | 1-12 |
| A | JP 2021-052655 A (FUJI OIL HOLDINGS INC.) 08 April 2021 (2021-04-08)<br>entire text, all drawings | 1-12 |
| A | WO 2022/071418 A1 (AMANO ENZYME INC.) 07 April 2022 (2022-04-07)<br>entire text, all drawings | 1-12 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **23 April 2024** | **14 May 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

INTERNATIONAL SEARCH REPORT
Information on patent family members

International application No.

**PCT/JP2024/005385**

| Patent document cited in search report | | | Publication date (day/month/year) | Patent family member(s) | | | Publication date (day/month/year) |
|---|---|---|---|---|---|---|---|
| WO | 2022/063833 | A1 | 31 March 2022 | US | 2023/0413873 | A1 | |
| | | | | AU | 2021350081 | A | |
| | | | | BR | 112023004624 | A | |
| | | | | CN | 116723768 | A | |
| | | | | CL | 2023000781 | A | |
| CN | 115669852 | A | 03 February 2023 | (Family: none) | | | |
| WO | 2022/138872 | A1 | 30 June 2022 | EP | 4268602 | A1 | |
| | | | | CN | 116648144 | A | |
| JP | 2021-052655 | A | 08 April 2021 | WO | 2021/066005 | A1 | |
| | | | | CN | 114449903 | A | |
| WO | 2022/071418 | A1 | 07 April 2022 | CN | 114304276 | A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014123466 A
- WO 2022144452 A
- WO 2022102723 A
- JP 2000050887 A
- JP 2001218590 A
- WO 2006075772 A1
- WO 2015133590 A

**Non-patent literature cited in the description**

- *Journal of Japan Society of Nutrition and Food Science*, vol. 20 (4), 259-266
- **BRODKORB et al.** *Nat. Protoc*, 2019